Europäisches Patentamt

European Patent Office

Office européen des brevets

(11) Veröffentlichungsnummer: **0 417 286 A1**

# (12) EUROPÄISCHE PATENTANMELDUNG
## veröffentlicht nach Art. 158 Abs. 3 EPÜ

(21) Anmeldenummer: 89909263.9

(22) Anmeldetag: 17.03.89

(86) Internationale Anmeldenummer:
PCT/SU89/00067

(87) Internationale Veröffentlichungsnummer:
WO 90/11061 (04.10.90 90/23)

(51) Int. Cl.5: **A61F 2/16**

(43) Veröffentlichungstag der Anmeldung:
20.03.91 Patentblatt 91/12

(84) Benannte Vertragsstaaten:
**DE FR GB IT**

(71) Anmelder: **MEZHOTRASLEVOI NAUCHNOTEKHNICHESKY KOMPLEX "MIKROKHIRURGIA GLAZA"**
**Beskudnikovsky bulvar, 59a**
**Moskau(SU)**

Anmelder: **INSTITUT PROBLEM MEKHANIKI AKADEMII NAUK SSSR**
**pr. Vermadskogo, 101**
**Moscow, 117526(SU)**

(72) Erfinder: **FEDOROV, Svyatoslav Nikolaevich**
**ul. Dostoevskogo, 21-37**
**Moscow, 103030(SU)**
Erfinder: **KOROSTELEVA, Natalya Fedorovna**
**pr. Mira, 11-23**
**Moscow, 129030(SU)**
Erfinder: **EGOROVA, Eleonora Valentinovna**
**ul. Kuusinena, 15-41**
**Moscow, 125252(SU)**
Erfinder: **SUSHKOVA, Natalya Alexandrovna**
**p/o Arkhangelskoe, 2-19 Krasnogorsky raion**
**Moskovskaya obl., 143420(SU)**
Erfinder: **OSIPENKO, Nikolai Mikhailovich**
**ul. Mishina, 38/40-12**
**Moscow, 125083(SU)**
Erfinder: **DASHEVSKY, Ilya Nikolaevich**
**ul. Akademika Vinogradova, 6-210**
**Moscow, 117133(SU)**
Erfinder: **GOLDSHTEIN, Robert Velyanovich**
**pr. Vernadskogo, 99-1-240**
**Moscow, 117526(SU)**

(74) Vertreter: **Patentanwälte Zellentin & Partner**
**Zweibrückenstrasse 15**
**W-8000 München 2(DE)**

(54) **KRISTALLINE LINSE FÜR KRISTALLAUGE.**

(57) Eine künstliche Augenlinse enthält einen optischen Teil (1) und deren diametral zueinander angeordnete, symmetrische Abstützungselemente (2) mit Bogenform. Jedes bogenförmige Abstützungselement (2) weist in seinem Mittelteil einen Mittelabschnitt (5) auf, der dessen Schleifenäste (4) miteinander unter Ermöglichung deren gegenseitiger Verschwenkung in der Ebene des bogenförmigen Abstützungselementes (2) vereint.

Dieser Mittelabschnitt (5) ist als eine Windung (6) einer Schraubenspirale ausgeführt, die dem optischen Teil (1) zugekehrt ist.

EP 0 417 286 A1

FIG.1

# KÜNSTLICHE AUGENLINSE

Die vorliegende Erfindung bezieht sich auf das Gebiet der Medizin und insbesondere auf künstliche Augenlinsen, die in der Ophthalmologie zur Behandlung von Katarakten einer beliebigen Ursache in Frage kommen.

## Stand der Technik

Es ist eine künstliche Augenlinse bekannt, die eine eigentliche Linse als optischer Teil und daran diametral zueinander angeordnete symmetrische Abstützungselemente mit Bogenform, beispielsweise in Form von Schleifen, (Ophthalmology, 1982, vol.89. Nr. 8. S. 128. model PC-60),aufweist deren Astenden am optischen Teil befestigt sind.

Bei dieser Ausführung der künstlichen Augenlinse zwecks deren besserer Festlegung innerhalb des Auges eines Kranken und Aufrechterhaltens deren Stellungslage innerhalb des ausgewählten Augenbereiches ohne Versetzungen und Verformungen beim Gebrauch ist eine erhebliche Starrheit der fertiggestellten Abstützungselemente der Augenlinse vorgesehen.

Jedoch gibt eine nicht genügende Elastizität der Abstützungselemente keine Möglichkeit, deren Abmessungen im Laufe der jeweiligen Implantation wesentlich zu verändern, wodurch jeweils die Durchführung der Operationen erschwert und deren Dauer verlängert wird. Eine starre Gestaltung der Abstützungselemente kann ausserdem eine starke Beschädigung des Pigmentblattes der Regenbogenhaut und der Zellen des Hinterepithels der Hornhaut unter nachfolgender Entwicklung von Nachoperationskomplikationen wie sekundäres Glaukom, chronische Iridozyklitis bewirken: ein grosser Durchmesser der Linse selbst bedarf speziellen Zubehörs für die Implantation, wodurch die Zeitdauer der jeweiligen Operation auch verlängert wird, es müssen mehrere künstlichen Augenlinsen mit dem Durchmesser des Kapselsacks der Augenlinse vergleichbaren Ausmaßes hergestellt werden.

## Offenbarung der Erfindung

Der vorliegenden Erfindung liebt die Aufgabe zugrunde, eine künstliche Augenlinse mit derartiger Ausbildung der Abstützungselemente anzugeben, die es möglich macht, deren Ausmaße auch während der Implantation zu verändern und zu gleicher Zeit ihre für die eindeutige Festlegung der künstlichen Augenlinse innerhalb eines ausgewählten Bereiches des Auges des Kranken notwendige Starrheit beizubehalten und eine Verkürzung der Zeitdauer der Implantation unter gleichzeitiger Herabsetzung der Verletzungsgefahr während des Operationsablaufs zu gewährleisten.

Die gestellte Aufgabe wird dadurch gelöst, dass in einer künstlichen Augenlinse, die einen optischen Teil und daran diametral zueinander angeordnete, symmetrische Abstützungselemente mit Bogenform einschliesst, deren Astenden am optischen Teil befestigt sind, erfindungsgemäss jedes bogenförmige Abstützungselement in seiner Mitte einen Abschnitt aufweist, der dessen Äste miteinander unter Ermöglichung ihrer gegenseitiger Verschwenkung in der Ebene des jeweilien, bogenförmigen Abstützungselementes vereint.

Gemäss einer Ausführungsvariante der Erfindung ist der die Äste eines jeweiligen Abstützungselementes miteinander vereinende Mittelabschnitt als eine Windung einer Schraubenspirale ausgebildet, welche dem optischen Teil zugewandt ist.

Das Wesen der Erfindung besteht darin, dass die künstliche Augenlinse einen optischen Teil und zwei bogenförmige, symmetrische, einander diametral gegenüber-liegende Abstützungselemente vorsieht, von denen jedes durch am optischen Teil befestigte Schleifenäste gebildet ist und in der Mitte je einen die Schleifenäste miteinander unter Ermöglichung deren gegenseitiger Verschwenkung in der Ebene des jeweiligen Abstützungselementes vereinenden Abschnitt aufweist.

Einer weiteren Ausführungsvariante der vorliegenden Erfindung gemäss ist der die Schleifenäste miteinander vereinende Abschnitt als zumindest eine Ausnehmung ausgebildet, die auf der Aussenseite des jeweiligen Abstützungselementes ausgespart ist.

Vorzugs weise ist zumindest eine solche Ausnehmung an der jeweiligen Windung der Schraubenspirale aussenseitig auszusparen.

Durch die Windung der Schraubenspirale bzw. zumindest einer Ausnehmung an dem Mittelabschnitt des jeweiligen Abstützungselementes wird dessen Starrheit vermindert und auch die Möglichkeit erreicht, beim Ergreifen des Abstützungselementes mit der Pinzette bei der Implantation den Mittelabschnitt des Abstützungselementes näher in Richtung auf den optischen Teil der künstlichen Augenlinse zu verstellen, wodurch sich die Schleifenenäste des betreffenden Abstützungselementes einander entgegen unter ihrer Verschwenkung in Bezug auf ihre festeingeklemmten Enden in der Ebene des bogenförmigen Abstützungselementes verstellen.

Durch diese Bewegung der Schleifenäste werden die Ausmaße des jeweiligen Abstützungselementes kleiner, wobei die Verkleinerungsgrösse der Abmessungen des jeweiligen Abstützungselementes je nach den dem Chirurgen bevorstehenden, konkreten Aufgaben und Besonderheiten des Operationsauges eingestellt werden kann.

Außer den obenbeschriebenen Vorteilen bringen die erwähnten Ausführungsvarianten des die Schleifenäste des jeweiligen Abstützungselementes vereinigenden geeignete Abschnitts Verhältnisse zum Ergreifen des Abstützungselementes bei der Implantation mit der Pinzette mit sich, vereinfachen die chirurgische Technik bei der Einführung der Abstützungselemente ins Innere des Kapselsacks bzw. in die Ziliarfurche sowie vermeiden die sonst möglichen Verletzungen des Kapselsacks während der Implantation und vermindern die Verletzung des Pigmentblattes der Regenbogenhaut und der Zellen des Hinterepithels der Hornhaut. Durch die Benutzung der vorliegenden Erfindung werden Nachoperationskomplikationen wie Dezentrierung der künstlichen Augenlinse in Bezug auf die optische Achse des Auges, sekundäres Glaukom, Iridozyklite herabgesetzt.

Nachstehend wird die Erfindung anhand der Beschreibung von konkreten Ausführungsvarianten unter Bezugnahme auf Zeichnungen näher erläutert. Es zeigt:

Fig. 1 eine künstliche Augenlinse in Gesamtansicht:

Fig. 2 die Augenlinse gemäß Fig. 1 in Seitenansicht;

Fig. 3 einen Schleifenäste des Abstützungselementes vereinenden Abschnitt,

Fig. 4 eine weitere Ausführungsvariante des die Schleifenäste des Abstützungselementes vereinenden Abschnitts:

Fig. 5 eine andere Ausführungsvariante des die Schleifenäste des Abstützungselementes vereinenden Abschnitts.

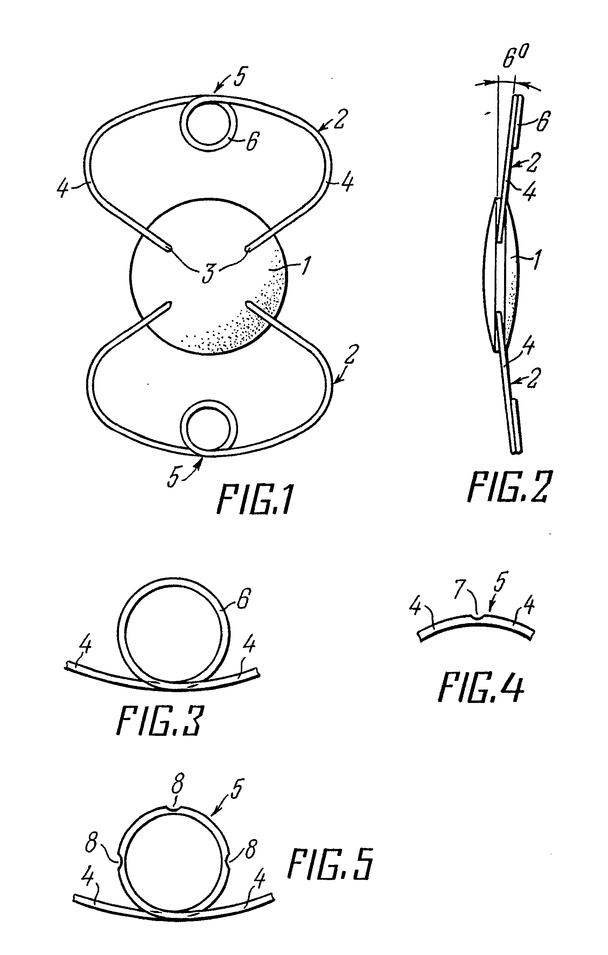

Eine künstliche Augenlinse enthält einen optischen Teil 1 (Fig. 1) und zwei symmetrische, diametral zueinander daran angeordnete Abstützungselemente 2 mit Bogenform, die mit den Enden 3 über Äste 4 in beliebiger, bekannter Weise am optischen Teil 1 befestigt sind. An jedem bogenförmigen Abstützungselement 2 ist in dessen Mitte ein Abschnitt 5 vorgesehen, der dessen Schleifenäste 4 unter Ermöglichung ihrer gegenseitigen Verschwenkung in der Ebene des bogenförmigen Abstützungselementes 2 vereint.

Der die Schleifenäste 4 miteinander vereinende Abschnitt 5 wird gemäss Fig. 1. 2. 3 als eine Windung 6 einer Schraubenspirale, die dem optischen Teil 1 zugekehrt ist, oder in Form zumindest einer Ausnehmung 7 (Fig. 4) ausgebildet, die auf der Aussenseite des Abstützungselementes 2 ausgespart ist, wie dies in Fig. 4 dargestellt ist.

Wie die Fig. 5 veranschaulicht, kann der die Schleifenäste miteinander vereinende Abschnitt 5, der als eine Windung 6 der Schraubenspirale ausgeführt ist. Ausnehmungen 8 auf der Aussenseite dieser Windung aufweisen Es können eine, zwei oder drei(wie in Fig. 5) Ausnehmungen 8 vorgesehen sein.

Die Implantation der erfindungsgemässen, künstlichen Augenlinse wird wie folgt durchgeführt.

Nachdem die Hornhaut an einem mit der Stellungslage des Uhrzeigers zwischen 11 und 13 Uhr übereinstimmenden Bereich zerschnitten ist, das Sehloch durch Einführung einer 1%igen Mesatonlösung ins Vorderkammerwasser erweitert wird, wird die vordere Kapseleröffnung,die Entfernung des Kernes und die Evakuierung der Linsenmasse durchgeführt. Nun wird die künstliche Augenlinse mit einer modifizierten Binkhorst-Pinzette ergriffen und mit dem unteren Abstützungselement unter einem Glätter in das untere Kapselgewölbe eingeführt. Im weiteren werden die Pinzette und der Glätter aus der Augenhöhle herausgenommen.

Jetzt wird die Windung 6 (Fig. 1, 3, 5) oder die Ausnehmung 7 (Fig. 4) mit der Pinzette ergriffen und der Mittelabschnitt des oberen Abstützungselementes 2 in Richtung auf den optischen Teil 1 (Fig. 1) verschoben, wodurch sich jeder der Schleifenäste 4 des jeweiligen Abstützungselementes 2 unter Bewegung auf einander zu in der Ebene des betreffenden Abstützungselementes in Bezug auf die festeingeklemmten Astenden 3 verschwenkt. In diesem Zusammenhang tritt eine Verminderung der Ausmaße des Abstützungselementes 2 auf, wobei bei dem in Form einer Windung 6 ausgebildeten Mittelabschnitt 5 diese Verkleinerung der Ausmaße durch eine Vergrösserung des Durchmessers dieser Windung während der Einander-Entgegenbewegung der Schleifenäste 4 und deren Biegung im Abstützungselement 2 und im in Form einer Ausnehmung 7 ausgeführten Mittelabschnitt 5 diese Verkleinerung nur durch Biegung der Schleifenäste 4 in Richtung auf den optischen Teil erreicht wird.

Hiernach wird das obere Abstützungselement 2 hinter das obere Blatt der Linsenvorderkapsel eingeschoben. Die Operation wird mit der hermetischen Abdichtung der Vorderkammer und mit der Wiederherstellung deren Tiefe bis auf 3.0 mm vermittels einer isotonischen Salzlösung beendet.

Dank den Elastizitätskraften ihrer Abstützungselemente, die eine gute Adaptation der ganzen Linsenoberfläche an den Umgebungsgeweben bewirken, kann die erfindungsgemäss ausgeführte, künstliche Augenlinse sowohl in den Kapselsack als auch in die Ziliarfurche implantiert werden.

Durch Verminderung der Anzahl der Operations- bzw. Nachoperationskomplikationen bei der Implantation der erfindungsgemässen, künstlichen Augenlinse wird eine Verkürzung der Fristen der medizinischen und beruflichen Rehabilitation der Kranken vermerkt. Somit ermöglicht die Benutzung der erfindungsgemässen, künstlichen Augenlinse die Zeitdauer bei der Durchführung der Implantation um 25% bis 30% zu verkürzen. Diese Verkürzung wird durch eine mit der aufbaumässigen Ausführung der künstlichen Augenlinse gesicherte Vereinfachung deren Implantationstechnik beim Einsetzen in den Kapselsack erreicht, wodurch auch die Verletzungsgefahr beim chirurgischen Eingriff herabgesetzt wird. Eine ausreichende Elastizität der Abstützungselemente und die Stabilität der Stellungslage der erfindungsgemässen, künstlichen Augenlinse innerhalb des Kapselsacks sowie deren einfache Implantation bewirken auch die Herabsetzung der Verletzungsstärke der Augengewebe während sowohl der Implantation selbst als auch der Nachoperationsperiode, was sich auch bei einer Verkleinerung der Beschädigung des Pigmentblattes der Regenbogenhaut und des Verlustes an Zellen des Hinterepithels der Hornhaut auswirkt.

Die erfindungsgemässe künstliche Augenlinse wird in der Ophthalmologie zur Behandlung von Katarakten einer beliebigen Herkunft eingesetzt, wobei deren Implantation sowohl in den Kapselsack als auch in die Ziliarfurche des Operationsauges durchgeführt werden kann.

**Ansprüche**

1.   Künstliche Augenlinse, die einen optischen Teil (1) und deren diametral gegenüberliegend angeordnete, symmetrische Abstützungselemente (2) mit Bogenform vorsieht, deren Enden (3) ihrer Schleifenäste (4) am optischen Teil (1) festeingeklemmt sind. dadurch **gekennzeichnet.** dass jedes bogenförmige Abstützungselement (2) in seinem Mittelteil einen Abschnitt (5) aufweist, der dessen Schleifenäste (4) miteinander unter Ermöglichung deren gegenseitiger Verschwenkung in der Ebene des jeweiligen, bogenförmigen Abstützungselementes (2) vereint.

2.   Künstliche Augenlinse nach Anspruch 1, dadurch **gekennzeichnet**, dass der die Schleifenäste (4) miteinander vereinende mittlere Abschnitt (5) als eine Windung (6)einer Schraubenspirale ausgebildet ist, die dem optischen Teil (1) zugekehrt ist.

3.   Künstliche Augenlinse nach Anspruch 1, dadurch **gekennzeichnet,** dass der die Schleifenäste (4) miteinander vereinende Abschnitt (5) in Form zumindest einer Ausnehmung (7) ausgeführt ist, die an jedem Abstützungselement (2) aussenseitig ausgespart ist.

4.   Künstliche Augenlinse nach Anspruch 2, dadurch **gekennzeichnet,** dass an der Windung (6) der Schraubenspirale aussenseitig zumindest eine Ausnehmung (8) ausgespart ist.

FIG.1

FIG.2

FIG.3

FIG.4

FIG.5

# INTERNATIONAL SEARCH REPORT

International Application No `PCT/SU89/00067`

## I. CLASSIFICATION OF SUBJECT MATTER (if several classification symbols apply, indicate all)

According to International Patent Classification (IPC) or to both National Classification and IPC

IPC⁵: A61F 2/16

## II. FIELDS SEARCHED

| Minimum Documentation Searched [7] | |
|---|---|
| Classification System | Classification Symbols |
| IPC⁴ | A61F 2/16, 9/00 |

Documentation Searched other than Minimum Documentation
to the Extent that such Documents are Included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| A | US, A, 4251887 (A.Y. ANIS)<br>24 February 1981,<br>see figures 3-5 | 1-4 |
| A | DE, AI, 3722910 (ADATOMED PHARMAZEUTISCHE UND MEDIZINTECHNISCHE GESELLSCHAFT MBH),<br>19 January 1989,<br>see figures 1,2 | 1-4 |
| A | US, A, 4536896 (T. BITTNER)<br>27 August 1985,<br>see figures 1,3 | 2 |
| A | US, A, 4446581 (L.W. BLAKE)<br>8 May 1984,<br>see figure 3 | 2 |
| A | US, A, 4624670 (C.H. BECHERT)<br>25 November 1986, | 3 |
| A | WO, AI, 86/00520 (FEASTER F.T.)<br>30 January 1986, | 2-4 |

./.

## IV. CERTIFICATION

| Date of the Actual Completion of the International Search | Date of Mailing of this International Search Report |
|---|---|
| 23 November 1989 (23.11.89) | 6 December 1989 (06.12.89) |
| International Searching Authority | Signature of Authorized Officer |
| ISA/SU | |

Form PCT/ISA/210 (second sheet) (January 1985)